# EUROPEAN PATENT APPLICATION

(11) **EP 4 478 378 A1**
(43) Date of publication of application: **18.12.2024**
(21) Application number: 22932369.6
(22) Date of filing: 23.03.2022
(51) Int. Cl.: G16H 80/00, G16H 50/20, G16H 50/70, A61B 34/20, G06F 3/00, G06F 3/01, G06F 3/16, G06T 19/00

(54) **REMOTE COLLABORATION SUPPORT SYSTEM IN WHICH REAL-TIME SURGICAL VIDEO CAN BE TAGGED**

(30) Priority: 17.03.2022 KR 20220033105
(71) Applicant: MediThinQ, Gyeonggi-do 13486 (KR)
(72) Inventor: PARK, Soyun, Seongnam-si, Gyeonggi-do 13325 (KR); KIM, Seonghoon, Seongnam-si, Gyeonggi-do 13375 (KR); JUNG, Yoonsang, Seongnam-si, Gyeonggi-do 13385 (KR)
(74) Representative: Manasse, Uwe
(86) International application number: PCT/KR2022/004040
(87) International publication number: WO 2023/177002

(57) **Abstract**

The present invention relates to a remote collaborative treatment support system in which a real-time surgical video can be tagged. More particularly, the remote collaborative treatment support system according to the present invention comprises, a first HMD worn by an operating surgeon at an actual surgical operation site, and including a camera for imaging a gazing area and an adjacent area of the operating surgeon and a display unit for outputting a realtime video captured by the camera. And a second HMD, which is worn on a collaborating surgeon located remotely from the operating surgeon, receives and outputs the realtime video captured in the first HMD, and receives, from the collaborating surgeon, a proposal for the order of surgical operation or a specific treatment sequence included in the surgical operation. And a remote collaborative treatment providing server which provides a UI for receiving the selection of whether to accept the proposal through the first HMD when the proposal input from the second HMD is received, and which generates a tag according to whether the operating surgeon has accepted so that same is tagged onto a collected real-time video, wherein the tag includes responsibility party information that shows person-in-charge about the realtime surgical operation according to the proposal.

## Description

### [Technical Field]

The present invention relates to a system for providing a remote collaborative treatment for tagging a realtime surgical video, and more particularly, to a system for providing a remote collaborative treatment for tagging a realtime surgical video, which tags, when a collaborative treatment proposal by a collaborating surgeon occurs during a realtime surgery, a responsibility according to whether an operating surgeon accepts the collaborative treatment proposal to a realtime video to use the video for determining the responsibility in the event of a medical accident.

### [Background Art]

In recent years, as multimedia related technology has developed radically and has been used in various fields, even in the medical field, technology has been developed to produce recorded videos of patients' surgery scenes so that doctors can present the scenes at medical conferences or use the scenes for other purposes.

In addition, surgical videos are recorded to record the surgery and to defend against any medical disputes that may arise, and since most medical equipment is equipped with a video recording function, the recording of surgical videos is rapidly increasing.

Also, depending on the size of the hospital and the disease, surgery is often performed through collaboration with various specialists. However, due to time/space constraints, remote collaborative treatments are being conducted through messages, videos, etc.

When the surgery is performed through such a remote collaborative treatment, it is difficult to determine the details of the collaborative treatment in the surgical video, so a method is needed to distinguish a responsibility in the surgical video during the remote collaborative treatment.

In addition, during the remote collaborative treatment, it is necessary to provide the collaborating surgeon with a real-time reproduction of the atmosphere of a surgical site, and when suggesting the collaborative treatment, the surgical video must be shared with the operating surgeon through accurate pointing. In particular, when pointing to a patient's affected area, pointing is difficult inside the body. Accordingly, a method that allows three-dimensional pointing on the realtime surgical video shared by the collaborating surgeon with the operating surgeon is required.

Further, the recorded surgical videos are valuable for various uses. In particular, when various medical information is added to the surgical video, the surgical video has utility and asset value, so a system that can broker stable transactions is needed.

### [Related Art Document]

(Patent Document 1): Korean Patent Unexamined Publication No. 10-2020-0001266 (Published on January 6, 2020)

### [Disclosure]

### [Technical Problem]

A technical object to be achieved by the present invention is to provide a system for providing a remote collaborative treatment for tagging a realtime surgical video, which tags, when a collaborative treatment proposal by a collaborating surgeon occurs during a realtime surgery, a responsibility according to whether an operating surgeon accepts the collaborative treatment proposal to a realtime video to use the video for determining the responsibility in the event of a medical accident.

However, technical objects of the present invention are not restricted to the technical object mentioned as above. Unmentioned technical objects will be apparently appreciated by those skilled in the art by referencing the following description.

### [Technical Solution]

In order to achieve the problem, a system for providing a remote collaborative treatment according to an embodiment of the present invention may include: a first HMD worn by an operating surgeon at an actual surgery site, and including a camera capturing a gaze area of the worn operating surgeon and a surrounding area, and a display unit outputting a realtime video captured by the camera; a second HMD worn by a collaborating surgeon positioned remotely from the operating surgeon, receiving and outputting the realtime video captured by the first HMD, and receiving a proposal for a surgery progress sequence or a specific treatment sequence include in a surgery by the collaborating surgeon; and a remote collaborative treatment providing server providing a UI for selecting whether to accept the proposal to the first HMD upon receiving the input proposal from the second HMD, and generating a tag according to whether the operating surgeon accepts the proposal and tagging the generated tag to the collect realtime video.

In this case, the tag may include responsibility party information for a realtime surgery according to the proposal.

Further, the tag may further include tagging location information in the realtime video, and the information included in the tag may be stored in a blockchain-based distribution ledger.

Further, when receiving a preset specific input from the first HMD, the remote collaborative treatment providing server may generate a timestamp and tags the timestamp to the realtime video, and divide the real-time surgery into a plurality of detailed sections defined by the operating surgeon based on a plurality of tagged timestamps.

In this case, the tagging location information may include a start timestamp and an end timestamp of a detailed section including a tagging point of the corresponding tag.

Further, the preset specific input may become at least one of a gesture, a voice command, and a control signal command, and the detailed section may be a section corresponding to a detailed treatment and a surgery sequence of the realtime surgery defined by the operating surgeon.

In addition, the remote collaborative treatment providing server may provide a preview of the proposal to the first HMD jointly with the UI, and insert AR data corresponding to the proposal into the realtime video, and display the realtime video inserted with the AR data to the first HMD and the second HMD when the proposal is accepted through the first HMD.

In addition, the remote collaborative treatment providing server may provide, to the first HMD and the second HMD, an avatar of a collaborating surgeon which automatically performs a preset collaborating surgeon signature for a proposal responsibility jointly with the proposal when the proposal by the collaborating surgeon is received from the second HMD, and provide, to the first HMD and the second HMD, an avatar of an operating surgeon automatically performing a preset operating surgeon signature for a proposal acceptance responsibility when accepting the proposal in the first HMD.

Further, the remote collaborative treatment providing server may be automatically set with a responsibility agreement for a specific treatment or surgery predefined for each surgery category and patient condition from each of the first HMD and the second HMD, and provide the first and second HMDs with an avatar that automatically performs the preset signature of the corresponding HMD for which responsibility agreement is automatically set when a proposal for a specific treatment or surgery predefined for each HMD occurs.

In addition, the system for providing a remote collaborative treatment may further include an AI-based recommendation server automatically proposing tags included in the detailed section to the first HMD when a detailed section included in a similar surgery similar to the realtime surgery among a DB storing a surgery video tagged with at least one tag and surgery videos prestored in the DB corresponds to a predefined specific treatment or surgery in which the responsibility agreement is set by the second HMD.

In addition, the remote collaborative treatment providing server may automatically tag, when receiving the proposal for the predefined specific treatment or surgery in which the responsibility agreement is automatically set from the first HMD, the proposal to the realtime video.

Further, the system for providing a remote collaborative treatment may further include an AI-based recommendation server automatically proposing, when a detailed section included in a similar surgery similar to the realtime surgery among prestored surgery videos corresponds to the predefined specific treatment or surgery in which the responsibility agreement is set by the first HMD, at least one tag included in the detailed section of the similar surgery to the first HMD.

Further, the remote collaborative treatment providing server may tag at least one tag automatically proposed by the AI-based recommendation server to the realtime video.

In addition, the second HMD may selectively output at least one of a first screen that outputs the realtime video captured by the first HMD, and a second screen that displays at least one proposal which may be made during the realtime surgery as a preview window.

Further, the system for providing a remote collaborative treatment may further include a DB classifying and storing the surgery video tagged with at least one tag for each doctor, for each patient condition, and for each treatment and surgery category, and the second HMD may set at least one of the tags tagged to the surgery video stored in the DB as an interested tag, and is set with a display time and a display interval of the interested tag, and the remote collaborative treatment providing server may output the interested tag to a preview window of the second screen according to settings of the second HMD.

In addition, the system for providing a remote collaborative treatment may further include the AI-based recommendation server recommending the tags tagged to the similar surgery similar to the realtime surgery as the preview window from a previous surgery video of the collaborating surgeon.

Further, the system for providing a remote collaborative treatment may further include an AI-based recommendation server automatically providing, when the tag tagged upon the similar surgery similar to the realtime surgery is not proposed during the realtime surgery among the previous surgery videos performed by the collaborating surgeon, the tag tagged upon the similar surgery to the first HMD by the proposal of the collaborating surgeon.

In addition, the AI-based recommendation server may determine a tagging point and a tagging interval of the tag proposed in the realtime video, and automatically propose the tag to the first HMD, based on a tagging point and a tagging interval of the tag tagged upon the similar surgery.

Further, based on third HMDs worn by a plurality of assistants, respectively during the realtime surgery at the actual surgery site, a DB storing the surgery video tagged with at least one tag, and role information of a wearer for each HMD for the realtime surgery, a tag requiring essential playback may be extracted in each HMD, and automatically proposed to a corresponding HMD.

Details of other embodiments will be included in the detailed description and the drawings.

### [Advantageous Effects]

According to embodiments of the present invention, a system for providing a remote collaborative treatment for tagging a realtime surgical video can provide a remote collaborative treatment during a realtime surgery, and tag an event such as a collaborative treatment proposal which occurs during the realtime surgery to a realtime video, and store and utilize a surgical video.

In particular, when a collaborative treatment proposal by a collaborating surgeon occurs during the realtime surgery, responsible party information is tagged to the realtime video according to whether an operating surgeon accepts the collaborative treatment proposal to use the realtime video for determine a responsibility in the event of a medical accident.

In addition, through a head mounted display (HMD) of the remotely located collaborating surgeon, a captured field of view based on each camera at an actual surgery site is provided in a fixed field of view mode to add augmented reality, and a viewing field fixation release mode is provided so that the collaborating surgeon can independently determine the status of the surgery.

In addition, through a 3D pointing system that allows 3D pointing on the realtime video upon the collaborative treatment proposal, the collaborating surgeon can point in 3D to the patient's affected area, i.e., the inside of the body, that is needed during the collaborative treatment, and share a medical video for the affected area with the operating surgeon.

In addition, by recording tag information in a blockchain ledger and brokerage use and transactions by granting NFT-based ownership to the surgical video containing tags, transparent and reliable transactions can be brokered and transaction profits can be distributed.

In addition, through AI-based recommendations, an optimal surgical video and optimal tags can be provided according to a playback purpose of a user (operating surgeon, collaborating surgeon, assistant 1, assistant 2, or trainee), and the utilization of the surgical video can be increased.

The effects according to the present invention are not limited by the contents exemplified above, and other various effects are included in this specification.

### [Description of Drawings]

FIG. 1 is a diagram describing a system for providing a remote collaborative treatment according to the present invention.
FIG. 2 is a block diagram describing a configuration of a head mounted display (HMD) of FIG. 1.
FIG. 3 is a configuration block diagram describing a configuration of a server for providing a remote collaborative treatment of FIG. 1.
FIGS. 4 and 5 are diagrams describing a remote collaborative treatment of a second HMD of FIG. 1.
FIG. 6 is a block diagram describing a configuration of a 3D pointing system according to an embodiment of the present invention.
FIG. 7 is a flowchart describing a 3D pointing control method according to an embodiment of the present invention.
FIG. 8 is a diagram describing a control device of a second HMD according to a first embodiment of the present invention.
FIG. 9 is a diagram describing a control device of a second HMD according to a second embodiment of the present invention.
FIG. 10 is a diagram describing a control device of a second HMD according to a third embodiment of the present invention.
FIG. 11 is a diagram describing tag section setting according to an embodiment of the present invention.
FIG. 12 is a diagram describing a preview window for a collaborative treatment proposal according to an embodiment of the present invention.
FIG. 13 is a diagram describing a display screen of a first HMD receiving the collaborative treatment proposal according to an embodiment of the present invention.
FIG. 14 is a diagram describing authentication according to a proposal responsibility and a proposal acceptance responsibility according to an embodiment of the present invention.
FIG. 15 is a block diagram describing a configuration of a video brokerage server of FIG. 1.
FIG. 16 is a diagram describing a blockchain structure of a system for providing a remote collaborative treatment.

### [Mode for Invention]

The following contents illustrate only a principle of the present invention. Therefore, those skilled in the art may implement the principle of the present invention and invent various apparatuses included in the concept and the scope of the present invention although not clearly described or illustrated in the present specification. In addition, it is to be understood that all conditional terms and embodiments mentioned in the present specification are obviously intended only to allow those skilled in the art to understand a concept of the present invention in principle, and the present invention is not limited to embodiments and states particularly mentioned as such.

Further, in the following description, ordinal number expressions such as first, second, etc., are used for describing equal or independent objects and should be appreciated that there is no meaning of main/sub or master/slave in the order.

The above-mentioned objects, features, and advantages will become more obvious from the following detailed description associated with the accompanying drawings. Therefore, those skilled in the art to which the present invention pertains may easily practice a technical idea of the present invention.

The features of various embodiments of the present invention can be partially or entirely bonded to or combined with each other and can be interlocked and operated in technically various ways so as to be fully appreciated by those skilled in the art, and the embodiments can be carried out independently of or together in association with each other.

A system for providing a remote collaborative treatment according to an embodiment of the present invention relates to a system that may provide a remote collaborative treatment by a collaborating surgeon which is remotely positioned when a realtime surgery is performed by an operating surgeon at an actual surgery site.

In this case, it is necessary to determine a responsibility for the realtime surgery and collaborative treatment, and in the present invention, through a scheme of tagging a tag for determining a responsibility for a collaborative treatment proposal to a realtime surgery video which is captured in real time, the surgical video may be used for determining the responsibility in the event of a medical accident.

In addition, the remote collaborative treatment providing system according to the present invention may provide an AR video corresponding to the actual surgical site based on realtime video in order to provide an actual surgical site atmosphere to the collaborating surgeon which is remotely positioned, and configure a 3D pointing system capable of 3D pointing to a specific location of the realtime video for proposal and sharing.

In addition, the remote collaborative treatment providing system according to the present invention may be configured by a video brokerage system for utilizing a surgical video in which a tag is tagged to a video which is captured during a realtime surgery (utilization according to purposes of an operating surgeon, the collaborating surgeon, and educators/trainees) and a profit structure based on the utilization.

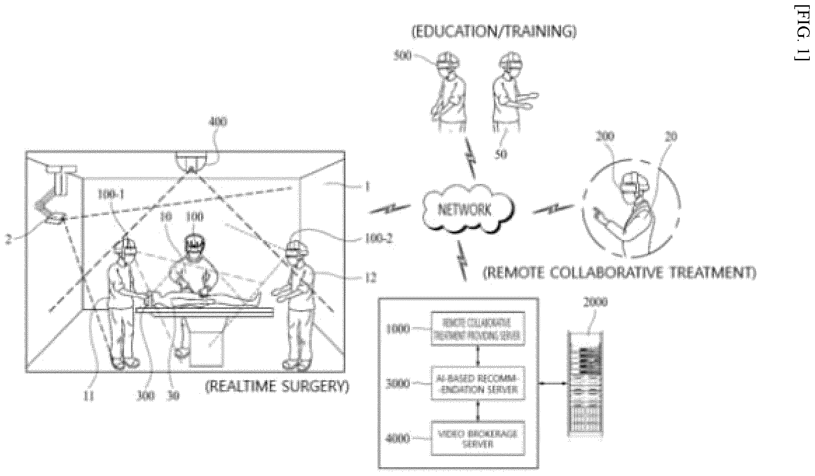

A detailed explanation of this will be provided with reference to FIGS. 1 to 14.

FIG. 1 is a diagram describing a system for providing a remote collaborative treatment according to the present invention.

The remote collaborative treatment providing system may be implemented with a first HMD 100 worn by an operating surgeon 10 and a second HMD 200 worn by a remotely located collaborating surgeon 20, and may include a remote collaborative treatment providing server 1000 for providing a remote collaborative treatment, a DB 2000, an AI-based recommendation server 3000, and a video brokerage server 4000.

Further, for providing the remote collaborative treatment for a realtime surgery at an actual surgery site 1, the realtime surgery is captured with a plurality of cameras to share a recorded video with the remotely located collaborating surgeon.

Specifically, a CCTV 2 is installed at the actual surgery site 1, and the remote collaborative treatment providing system may also further include third HMDs 100-1 and 100-2 worn by a plurality of assistants 11 and 12, a fourth HMD 300 worn by a patient 30, and a camera 400 installed on a ceiling of the surgery site 1 to provide a vivid ED image at the actual surgery site 1.

The camera 400 installed on the ceiling specifies coordinates for each object to determine an initial location in a first realtime video captured, and determines locations of the first HMD 100 worn by the operating surgeon 10, and the third HMDs 100-1 and 100-2 worn by a first assistant 11 and a second assistant 12 during the realtime surgery to perform movement tracking. Besides, movement of all objects such as surgical tools captured by the ceiling camera 400 may be tracked.

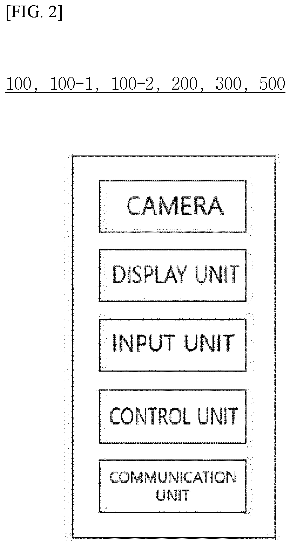

FIG. 2 is a block diagram describing a configuration of an HMD of FIG. 1.

Referring to FIG. 2, each of the HMDs 100, 100-1, 100-2, 200, 300, and 500 may include a camera, a display unit, an input unit, a control unit, and a communication unit, and further include a speaker, a microphone, etc., in addition thereto.

In this case, a video provided through on/off or a display unit of each component is applied differently according to a wearer, i.e., according to a wearer mode set to an operating surgeon mode, an assistant mode, a patient mode, an education/training mode, etc., as an example, to provide a function required for the corresponding wearer. Alternatively, the HMD for each wearer may also be constituted by separate HMDs providing only a function according to the corresponding mode.

In this case, the camera of the HMD may include a first camera provided in front of the HMD to capture a gaze area of the wearer, and second cameras provided on both sides of the HMD to capture a surrounding area of the wearer.

In addition, the display unit may display the video of the gaze area of the wearer captured by the camera in the operating surgeon mode and the assistant mode, and receive and display a realtime video of each camera captured at the actual surgery site in the collaborating surgeon mode. Besides, the patient mode may provide information as necessary, and the education/training mode may display a selected playback video.

The input unit may become an interface or a sensing unit for receiving control signals for controlling the HMD and controlling the displayed video, and the control unit may control the HMD according to the control signal received through the input unit, and receive various remote collaborative treatment supports from respective external servers 1000 to 4000 through the communication unit.

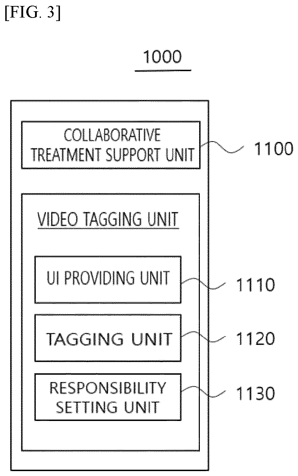

FIG. 3 is a block diagram illustrating a configuration of a remote collaborative treatment providing server 1000. The remote collaborative treatment providing server 1000 may include a collaborative treatment support unit 1100 and a video tagging unit 1200. In this case, each component may also be constituted by a separate module, a server, etc.

Here, the remote collaborative treatment support may be a realtime surgery related collaborative treatment proposal, determination of a responsibility according to the proposal video tagging of tagging to a video captured in real time when various events occur, 3D pointing control to a displayed video, utilization of a surgical video including a tag, and distribution of revenue for the utilization.

The collaborative treatment support unit 1100 collects realtime videos output by all cameras, which are captured in an operating room 1 so as for the collaborating surgeon to remotely participate in the collaborative treatment, and transmits the realtime video to the second HMD 200 to allow the collaborating surgeon to remotely participate in the collaborative treatment.

In this case, for remote participation of the collaborating surgeon, the collaborating surgeon should be possible to determine a situation in the operating room 1 independently of the operating surgeon through the worn second HMD 200.

Therefore, the second HMD 200 worn by the collaborating surgeon may provide the realtime video according to a capturing viewing field between the CCTV 2 installed in the operating room 1, the camera 400, the camera of the first HMD 100 worn by the operating surgeon, and the cameras of the third HMDs 100-1 and 100-2 worn by the plurality of assistants 11 and 12, respectively according to selection of the operating surgeon.

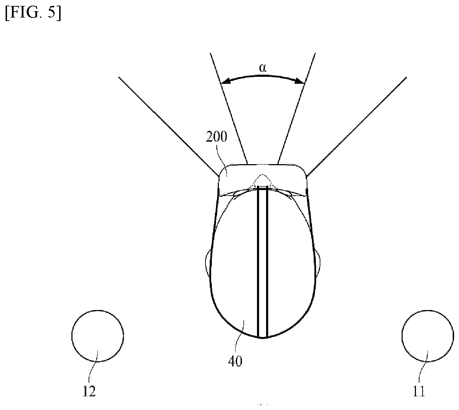

As an example, in the case of the second HMD 200 worn by the collaborating surgeon, when a specific location (as an example, the camera of the first HMD 100 worn by the operating surgeon as in FIG. 5) of the actual surgery site selected by the collaborating surgeon is selected through the display unit, the realtime video captured a first camera capturing viewing field of the first HMD 100 may be output to the display unit of the second HMD 200.

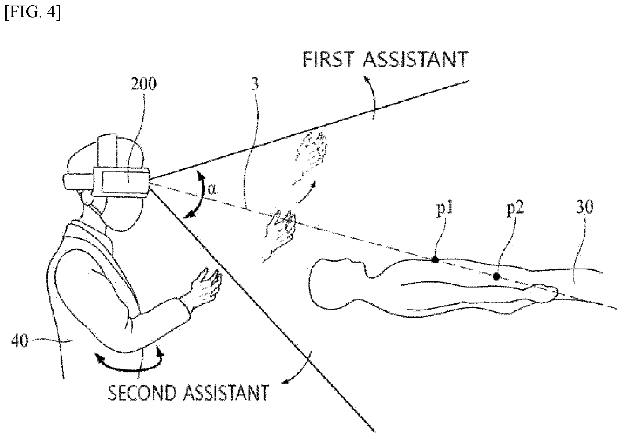

In this case, as in FIGS. 4 and 5, the collaborating surgeon may secure the same viewing field from the viewpoint of the operating surgeon, and after securing the viewing field, the collaborating surgeon turns his/her gaze independently of the operating surgeon to determine the situation in the operating room 1.

That is, in FIG. 4, when the collaborating surgeon turns the gaze in the direction of the first assistant 11 of the second assistant 2 12, a realtime video captured by a specific second camera corresponding to a gaze direction of the collaborating surgeon among the realtime videos captured at the capturing viewing fields of the second cameras provided at both sides of the first HMD 100 worn by the operating surgeon 10 may be output through the second HMD 200 of the collaborating surgeon 20. That is, since a surrounding is not fixed, the collaborating surgeon may move the viewing field independently of the operating surgeon (viewing field fixation release mode).

Besides, even when a specific camera is selected, the collaborating surgeon may similarly move the viewing field independently. Alternatively, only a realtime video captured by the selected camera may also be received by fixing the surrounding (viewing field fixation mode).

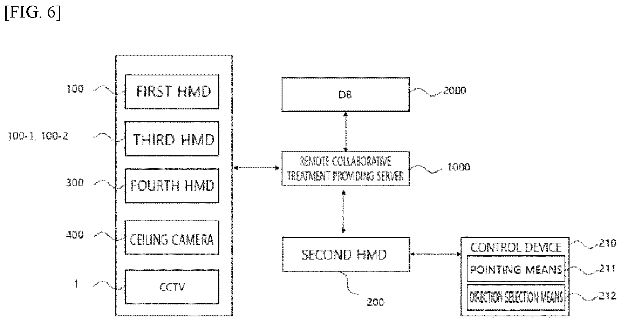

As such, in the present invention, the viewing field fixation mode may be provided according to the selection of the collaborating surgeon by the second HMD 200 worn by the collaborating surgeon 20 by fixing the surrounding. The collaborating surgeon may perform the collaborative treatment by 3D-pointing a location which needs to be shared for the collaborative treatment proposal through the remote collaborative treatment of FIG. 6 in the viewing field fixation mode through a 3D pointing system (hereinafter, referred to 3D pointing system).

### 3D pointing system for remote collaborative treatment

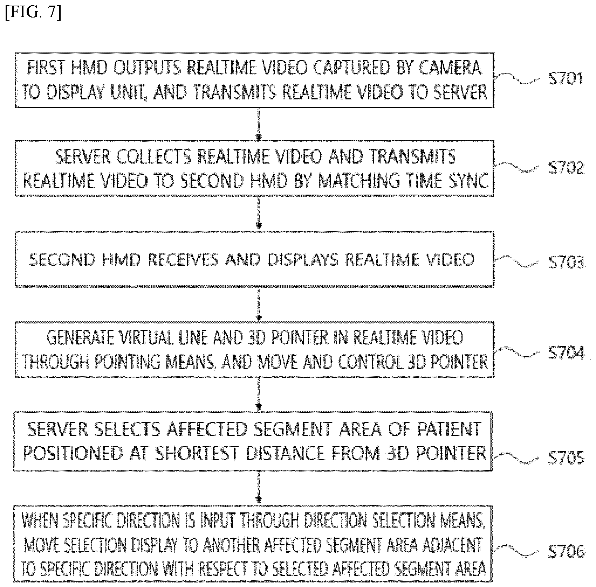

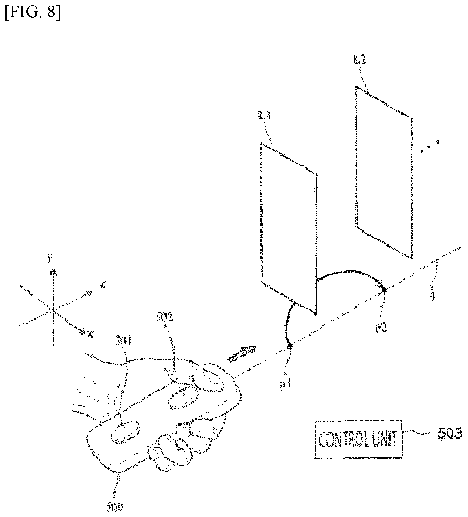

Referring to FIG. 6, the 3D pointing system according to an embodiment of the present invention may further include a control device 210 for controlling the second HMD 200 in the remote collaborative treatment providing system of FIG. 1, and the control device may be variously implemented as control devices 500, 500a, and 600 illustrated in FIGS. 8 to 10. The 3D pointing system is described jointly with a 3D pointing control method of FIG. 7.

Realtime videos captured by the first camera and the second camera of the first HMD 100 worn by the operating surgeon 10 which participates in a realtime surgery at the actual surgery site 1, the first camera and the second camera of the third HMDs 110-1 and 110-2 worn by the plurality of assistants 11 and 12, the camera of the fourth HMD 300 worn by the patient 30, the CCTV 2, and the ceiling camera 400 may be transmitted to the remote collaborative treatment providing server 1000.

In this case, the realtime video captured by the first camera may be output to the display unit of the first HMD 100, and transmitted to the remote collaborative treatment providing server 1000 (S701). Further, the realtime video captured by the first camera may be output to the display units of the respective third HMDs 110-1 and 110-2, and transmitted to the remote collaborative treatment providing server 1000.

Next the collaborative treatment support unit 1100 of the remote collaborative treatment providing server 1000 may collect the realtime video from each camera and synchronize a time sync, and transmit the video to the second HMD 200 (S702).

Next, the second HMD 200 may receive and display the realtime video of the first HMD 100 (S703), when the second HMD 200 receives the realtime videos of the plurality of HMDs 100, 100-1, and 100-2, the second HMD 200 may display a realtime video corresponding to a camera selected by the collaborating surgeon or a predetermined camera In this case, the realtime video of the first HMD 100 of the operating surgeon may be preferably output, and thereafter, may also be changed by the collaborating surgeon.

Next, the second HMD 200 may 3D-point a specific location for the collaborative treatment proposal to the realtime video output to the display unit according to the control signal received by the second HMD 200 through the input unit. In this case, the specific location may become a specific location in the actual operating room 1, and in particular, may become an affected area specific location of the patient for the collaborative treatment proposal. In the present invention, so as for the operating surgeon 10 and the collaborating surgeon 20 to share a treated affected area of the patient, and perform the collaborative treatment, the realtime video (the video generated by the first camera capturing the gaze area of the first HMD 100) needs to be set to the viewing field fixation mode in the second HMD 200. That is, the collaborating surgeon should be able to confirm the video from the viewpoint of the operating surgeon, and propose the surgery to the operating surgeon.

Referring to FIGS. 4, and 5, in the viewing field fixation mode, as in FIG 4, the second HMD 200 outputs only the realtime video output by the first camera of the first HMD 100 to the second HMD 200 to allow the collaborating surgeon to confirm a realtime surgery within a range corresponding to a capturing viewing field α of the first camera. That is, even though a movement (turning the head in the direction of the first assistant or the second assistant) of the collaborating surgeon occurs, only the realtime video in the capturing viewing field α is output.

Meanwhile, when a surrounding fixation release (viewing field fixation release mode) is set, a realtime video corresponding to a capturing viewing field of the second camera corresponding to a movement direction upon movement of the second HMD 200 is provided to confirm the surrounding by moving the viewing field independently of the operating surgeon.

As an example, in FIGS. 4 and 5, when the collaborating surgeon gazes at a first realtime video (a realtime video corresponding to the capturing viewing field α of the first camera of the first HMD 100) within the capturing viewing field α, and then turns the head, and turns the gaze in the direction of the first assistant 11, a second realtime video captured in the capturing viewing field of the second camera provided in the direction of the first assistant 11 of the first HMD 100 worn by the operating surgeon 10 is continuously provided to move the viewing field independently of the operating surgeon.

The 3D pointing control system according to the present invention may set the realtime video displayed in the second HMD 200 to the viewing field fixation mode, and then perform 3D pointing for the collaborative treatment proposal.

The control device 210 for 3D pointing may include a pointing means 211 and a direction selection means 212. The control device 210 may output a control signal for generating a virtual line and a 3D pointer in a 3D realtime video to the second HMD 200 according to the control of the operating surgeon, and control movement of the 3D pointer by using the pointing means 211 and the direction selection means 212 (S704). The pointing means 211 may move the 3D pointer in a depth direction (a z direction in FIGS. 8 to 10) along the virtual line generated in the 3D realtime video, and the direction selection means 212 may move the 3D pointer in a direction (an x or y direction in FIGS. 8 to 10) perpendicular to the depth direction.

The pointing means 211 and the direction selection means 212 may be variously implemented as the control devices 500, 500a, and 600 illustrated in FIGS. 8 to 10, and the direction selection means 212 may also be used as a foot pedal type.

Further, the control signal in step S704 may be a signal input by a predetermined gesture, gaze processing, eye movement (eye blinking, etc.) of the collaborating surgeon sensed by the input unit of the second HMD 200, or input by one of the control devices of FIGS. 8 to 10.

First, when a predetermined gesture, gaze processing, gaze movement of the collaborating surgeon sensed by the input unit of the second HMD 200 are used, as an example, the viewing field fixation mode may be set through a first gesture (a motion of opening both arms at a predetermined angle), a pointer P1 and a virtual line 3 may be formed in the affected area through the gaze processing (gazing at an affected area for pointing for a predetermined time or more), and a pointer P2 may be moved in the depth direction on the virtual line 3 through a second gesture (hand movement). This allows 3D pointing to the affected area inside the patient's body.

In this case, the depth may be set to be adjustable by matching a depth, and internal organs and blood vessels of the patient's body for each depth. More specifically, using the control device 210, the operating surgeon may select a specific affected segment area by moving the pointer in the depth direction z and the direction (x, y) perpendicular to the depth direction.

Specifically, the patient's affected segment area may be statistically determined by learning affected area video histories of other patients with the same medical history as the patient's affected area. In particular, the patient's affected segment area can be divided to reflect the affected area identified through indocyanine green (ICG) staining.

The ICG as a medical reagent has a feature in that the ICG fluoresces at an infrared wavelength of 805 nm as a green pigment and the ICG is bound to albumin, a water-soluble protein present in the body's cytoplasm and tissues. After the ICG is stained by injecting the ICG into the patient's blood vessels through intravenous injection, infrared rays are irradiated and the filtered reflected light is captured through a camera equipped with a filter to obtain a fluorescently stained video of the affected area.

The remote collaborative treatment providing server 1000 may collect videos of the affected area, learn the staining area for each patient's disease name, and statistically divide the affected segment area.

In addition, it can be divided into a plurality of sub-segments based on the blood vessel arrangement structure for the affected area, the arrangement structure of other surrounding organs, and the difference in depth of the affected area observed from each viewpoint of the operating surgeon and the collaborating surgeon.

Here, the difference in depth of the affected area observed from the viewpoint of each of the operating surgeon and the collaborating surgeon may be a difference in distance (depth level) in the depth direction z between the detailed affected area gazed by the operating surgeon and the detailed affected area gazed by the collaborating surgeon for one affected segment area.

On the other hand, when using an ICG-stained affected area video in the realtime surgery, the ICG-stained affected area video captured by the camera using the filter may be received from the first HMD and the second HMD and displayed by matching the ICG-stained affect area video with the realtime video.

Through this, the operating surgeon and the collaborating surgeon may perform the realtime surgery by selecting the affected segment area by 3D pointing. As an example, when the operating surgeon uses the pointing means 211 to generate the virtual line in the 3D realtime video and the 3D pointer positioned on the virtual line, the server 1000 may determine that select the patient's affected segment area located at the shortest vertical distance from the 3D location (x, y, z) of the 3D pointer, and provide a selection display to the affected segment area (S705). Accordingly, the operating surgeon and the collaborating surgeon may share the selection area through the first HMD 100 and the second HMD 200.

Further, the operating surgeon may move the 3D pointer in the vertical direction by selecting a specific direction signal in the vertical direction (x, y) with respect to the depth direction (z) using the direction selection means 212. That is, the server 1000 may move a selection display so that another affected segment area adjacent to the specific direction selected through the direction selection means 212 is selected based on the previously selected and displayed affected segment area (S706).

Further, the 3D pointing may also be provided through various embodiments of the control device in FIGS. 8 to 10 of the present invention.

### (First embodiment) - Control device 500

Referring to FIG. 8, the control device 500 may include a first input unit 501, a second input unit 502, and a control unit 503. In this case, the first input unit 501 and the second input unit 502 may be provided in the body in the form of a button.

When the first input unit 501 receives an input signal by button pushing, the first input unit 501 may output a first control signal for setting the realtime video output by the second HMD 200 to the viewing field fixation mode or the viewing field fixation release mode. The second HMD 200 may set the mode by receiving the first control signal.

Further, in a state in which the viewing field fixation mode is set by the first input unit 501, the first input unit 501 may output a second control signal for generating the virtual line 3 and a pointer p1 in the realtime video output by the second HMD 200 when the input signal is received by the button pushing.

Next, the control unit 503 outputs a third control signal of moving the pointer (p1 -> p2) on the virtual line 3 by sensing a body movement of the control device in a depth direction or a reverse depth direction of the virtual line to 3D-point to the realtime video displayed in the second HMD 200.

In this case, a selected layer L2 between layers L1 and L2 matched with the depth step of the pointed location may be selected and displayed. Here, the layer may become an affected area video acquired by dividing the 3D affected video into affected segment areas.

That is, the affected segment area at the shortest vertical distance in the depth direction (z) from the position of the 3D pointer P2 may be selected. Further, the 3D pointer is moved by moving the body of the control device 500 the direction (x, y) perpendicular to the depth direction (z), i.e., left and right or up and down based on a user's hand to select another affect segment area by using the direction selection means.

### (Second embodiment) - Control device 500a

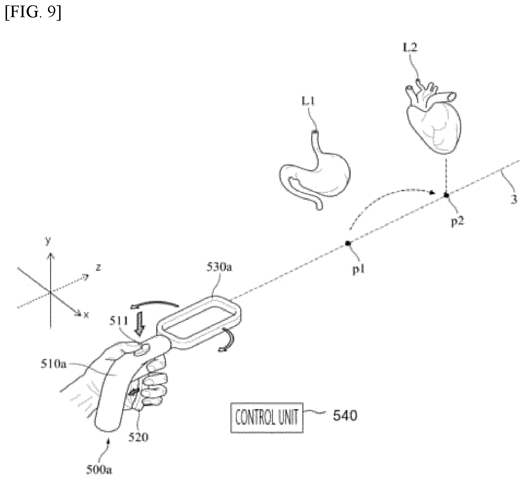

As another embodiment, the control device 500a of FIG. 9 in the form of a gun may improve user's grip feeling and convenience. Specifically, the control device 500a may include a handle part 510a including a header portion 530a, an input button 511, and a latch 520, and a control unit 540.

The header portion 530a may guide a direction so that a virtual line direction may point to a specific location by generating the virtual line in the realtime video when the input button 511 is pushed.

The input button 511 of the handle unit 510a may set the viewing field fixation mode when receiving an input signal and at the same time, generate the virtual line 3 and the pointer p1 using the header unit 530a. Further, the input button 511 re-pushed (the input signal is received again) in the state in which the viewing field fixation mode is set, the viewing field fixation release mode may be set.

The latch 520 of the handle unit 510a may output a control signal of moving the pointer on the virtual line 3 in the depth direction (p1-> p2) of the virtual line 3 whenever the latch is operated.

In this case, the affected segment area numbered to match a predetermined depth on the virtual line 3 may be provided in sequential circulation according to the depth whenever the latch is operated.

Further, the 3D pointer is moved by moving the body of the control device 500a in the direction (x, y) perpendicular to the depth direction (z), i.e., left and right or up and down based on a user's hand to select another affected segment area by using the direction selection means. Meanwhile, the control devices 500 and 500a may also be used only as the pointing means 211, and in this case, a separate foot pedal may be used as the direction selection means 212 to control the 3D pointing.

### (Third embodiment) - Control device 600

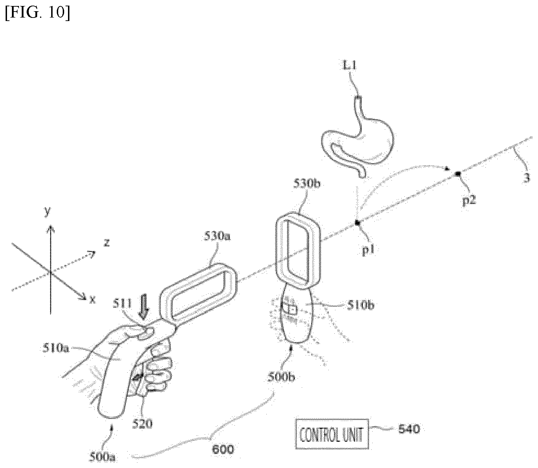

As yet another embodiment, the control device 600 of FIG. 10 is further equipped with a controller 500b in addition to the control device 500a of FIG. 9. That is, the control device 600 includes a first control device 500b and a second control device 500a. In this case, a form of the second control device 500a is the same as that in FIG. 9, but some functions may vary depending on settings.

The first control device 500b may include a handle 510b having a first button for setting the viewing field fixation mode and a second button for setting the viewing field fixation release mode, and a guide header unit 530b in which a guide ring is formed.

A virtual line generation direction by the header unit 530a may be guided by focusing the header unit 530a of the second control device 500a through the guide ring of the guide header unit 530b.

The input button 511 of the handle unit 510a may set the viewing field fixation mode when receiving an input signal and at the same time, generate the virtual line 3 and the pointer p1 using the header unit 530a. Further, the input button 511 re-pushed (the input signal is received again) in the state in which the viewing field fixation mode is set, the viewing field fixation release mode may be set.

The latch 520 of the handle unit 510a may output a control signal of moving the pointer on the virtual line 3 in the depth direction (p1-> p2) of the virtual line 3 whenever the latch is operated.

In this case, the affected segment area numbered to match a predetermined depth on the virtual line 3 may be provided in sequential circulation according to the depth whenever the latch is operated.

In this case, the latch 520 of the handle unit 510a may output a control signal of moving the pointer in the depth direction of the virtual line 3 (p1-> p2) whenever the latch is operated.

In this case, when the collaborative treatment support unit 1100 of the remote collaborative treatment providing server 1000 receives a specific pointer location (one location of the pointers such as p1, p2, etc.) on the virtual line 3 from the second HMD 200, the collaborative treatment support unit 1100 may insert and provide the affected segment area for an organ or blood vessel corresponding to the pointer location into the realtime videos output by the first to third HMDs. Further, the selected affected segment area L1 may also be divided into sub-segment areas and provided.

Meanwhile, the description focuses on the operating surgeon's 3D pointing, but the operating surgeon may 3D-point to the affected area in the same method.

### Video tagging of remote collaborative treatment providing system

The remote collaborative treatment providing system may tag to the realtime video captured by each camera, and thereafter, may be utilized for the surgery, collaborative treatment, education/training, and determination of the responsibility when the realtime surgery is in progress, a proposal for collaborative treatment generated during remote collaborative treatment, a recommendation proposal generated by the AI-based recommendation server 3000, and events directly set by the operating surgeon occur.

For this purpose, the remote collaborative treatment providing system is constituted by the remote collaborative treatment support server 1000, a DB 2000, an AI-based recommendation server 3000, and a video brokerage transaction server 4000, and referring to FIG. 3 the video tagging unit 1200 of the remote collaborative treatment support server 1000 may include a UI providing unit 1110, a tagging unit 1120, and a responsibility setting unit 1130.

Referring to FIG. 3, the remote participation of the collaborating surgeon and the realtime video tagging according to an embodiment of the present invention will be described.

In this case, the remote collaborative treatment providing system pre-sets priorities for cameras and captured videos that capture the realtime surgery, and displays captured videos in turn from the cameras in order according to the pre-set priorities on the display unit of the second HMD 200 to allow the collaborating surgeon to determine the collaborative proposal while monitoring. As an example, the collaborating surgeon may set or the collaborative treatment server 1100 may also preset the camera of the first HMD 100 of the operating surgeon, the CCTV, the first assistant, etc., in order.

Further, depending on the needs of the collaborating surgeon, a specific camera is selected to display the captured video.

Referring to FIGS. 1 to 3, the collaborating surgeon 20 may monitor the realtime surgical video the operating surgeon 10 through the second HMD 200 and transmit the proposal when the collaborative treatment is required.

The collaborative treatment support unit 1100 of the remote collaborative treatment providing server 1000 may collect the realtime video from each camera that captures the realtime surgery, and transmit the realtime video to the second HMD 200.

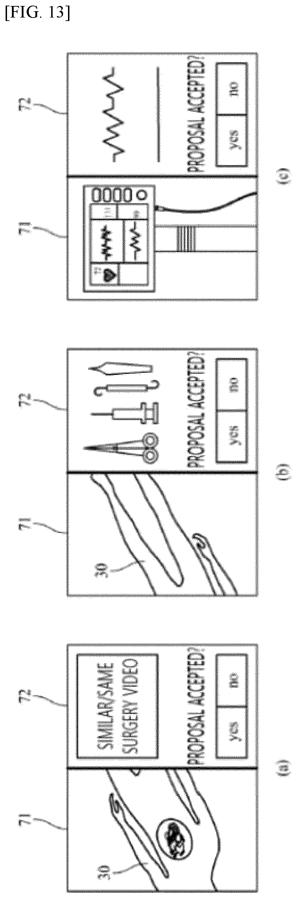

In this case, when the collaborative treatment proposal is received from the second HMD 200 or the proposal is recommended from the AI-based recommendation server 3000, the UI providing unit 1110 may provide a UI for selecting whether to accept the proposal to the first HMD 100. The UI for whether to accept the proposal may be provided as a text or provided as a voice as in FIG. 13.

The tagging unit 1120 may tag the realtime video according to the operating surgeon's acceptance when tags occur according to the proposal and events during the realtime surgery. In this case, the collaborating surgeon's proposal may be a proposal for a surgical procedure sequence or a specific treatment sequence included in the surgery. When acceptance of the proposal is received through the UI providing unit 1110, the tagging unit 1120 may generate a tag for the operating surgeon's acceptance from the first HMD 100 and tag the collected realtime video.

In this case, the tag may include information on a responsibility party for the realtime surgery according to the proposal and tagging location information within the realtime video, and the information included in the tag may be stored in a blockchain-based distribution ledger.

Here, the tag as metadata about the collaborating surgeon's proposal generated during the realtime surgery, a recommendation proposal generated by the AI-based recommendation server 3000, and events directly set by the operating surgeon may become marking information inserted into the realtime video.

As an example, the tag may include all information that may be an issue in the realtime surgery, such as the collaborating surgeon's AR surgery demonstration, collaborating surgeon's comments, previous surgery videos similar or identical to the realtime surgery, specific surgical tools, voice or text information, and vital signs.

In this case, the remote collaborative treatment providing system of the present invention may insert the responsibility party information for determining the responsibility when tagging the tag to the realtime video, and divide a tag section tagged to the realtime surgery into detailed section units divided into detailed treatment/surgery of the realtime surgery.

Specifically, when the tagging unit 1120 receives a preset specific input from the first HMD 100, the tagging unit 1120 may generate a timestamp and tag the timestamp to the realtime video, and divide the real-time surgery into a plurality of detailed sections defined by the operating surgeon based on a plurality of tagged timestamps.

Here, the preset specific input may be at least one of a gesture, a voice command, and a control signal command, and the detailed section may be a section corresponding to the detailed treatment and surgical sequence of the realtime surgery defined by the operating surgeon.

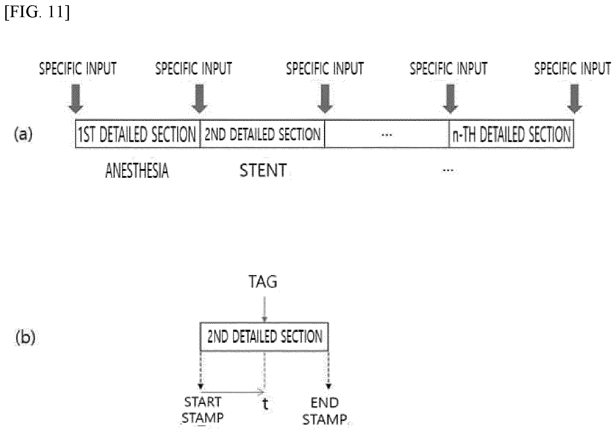

FIG. 11 is a diagram describing tag section setting according to an embodiment of the present invention. The operating surgeon 10 may provide information on the realtime surgery to the collaborating surgery 20 in advance.

In this case, the information provided in advance may be realtime surgical information, schematic detailed treatment, or surgical sequence. In this case, different collaborating surgeons may participate remotely for each detailed treatment, and the collaborative treatment support unit 1100 may limit the provision of the realtime video to the second HMD 200 of the corresponding collaborating surgeon 20 only during the detailed participation section.

In addition, for each detailed section during the realtime surgery, the operating surgeon 10 and the collaborating surgeon 20 may display a flag indicating that the detailed section for the surgery and treatment currently in progress is the section they are responsible for. In this case, in a state where one party raises the flag, the tag information of the corresponding detailed section may be marked as the responsibility section of one party.

In this case, when both parties raise the flags or lower both flags, a person who does not agree with this responsibility section may raise an objection message to the objection, and the collaborative treatment support unit 110 may support voice communication when a predetermined time elapses after the objection message is displayed.

In addition, when it is determined who is responsible for the detailed section, the AR providing unit 1130 may confirm the realtime responsibility section by providing a preset avatar of the responsible person to the realtime surgery video.

In addition, when the operating surgeon 10 lacks experience in the relevant surgery, the specialist may become the collaborating surgeon 20 and perform the remote collaborative treatment. In this case, the collaborative treatment support unit 1100 displays the collaborating surgeon's responsibility section on the realtime video, and give a priority to tagging with history tag information (metadata) linked with a recorded video section if the collaborating surgeon 20's evaluation numerical data for the specialist's experience or current surgery experience is greater than the predetermined value than the operating surgeon 10.

Referring to FIG. 11, for example, in the case of a coronary artery stent procedure, a procedure name (coronary artery stent), patient information, detailed treatment or sequence (anesthesia, stent, etc.) may be provided to a remotely participating collaborating surgeon, and detailed sections may be distinguished through a specific input from the operating surgeon 10 during the realtime surgery.

In this case, the tagging unit 1120 may insert a timestamp at the point when a specific input is input through the first HMD 100 and distinguish the start and end of the detailed section.

In this case, since both hands are not free depending on the surgical procedure, it is desirable that the specific input becomes a preset gesture or voice input, and the setting of the detailed section may be distinguished through tags insertion or AI analysis after real-time video capturing. Further, it may be input as the voice of an assistant who participates at the actual surgery site 1 rather than the operating surgeon 10.

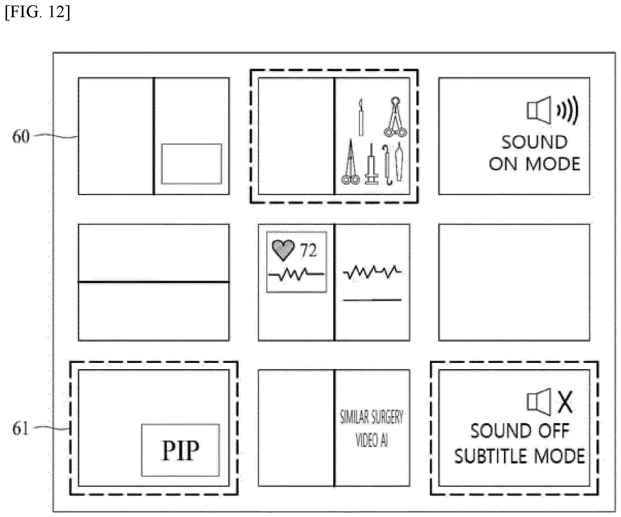

When the UI providing unit 1110 receives a proposal from the second HMD 200 or the AI-based recommendation server 3000, the UI providing unit 1110 may provide a preview of the collaborative treatment proposal along with the UI to the first HMD 100, as in FIG. 12(a).

FIG. 12 is a diagram describing a display screen of a first HMD receiving the collaborative treatment proposal according to an embodiment of the present invention.

Referring to FIG. 12, (a) illustrates a screen output on the first HMD 100 of the operating surgeon, which may display a realtime video 71 captured in real time by the first HMD 100, and a UI 72 inquiring about collaborative treatment proposed preview proposal contents and proposal acceptance.

Further, (b) may illustrate a screen output to the third HMD 100-1 of the first assistant when receiving the proposal of the collaborating surgeon, and (c) may illustrate a screen output to the third HMD 100-1 of the second assistant when receiving the proposal of the collaborating surgeon. In this case, the proposals received by the first and second assistants may become information received according to the proposal acceptance of the operating surgeon. That is, the proposals for (b) and (c) may also be displayed on the HMD 100 of the operating surgeon, and the first and second assistants may perform assistance according to the proposal accepted by the operating surgeon. That is, the operating surgeon may determine whether to accept the proposal according to the event occurrence.

That is, as in FIG. 12(b), the collaborating surgeon in a remote location may set the `virtual surgical tools' required for the surgery and display the setting on the HMDs of surgery participants 10, 11, and 12, and the surgery participants may prepare 'actual surgical tools' in advance while looking at the setting.

In addition, the collaborating surgeon may propose surgical tools to be used by the operating surgeon by mapping virtual surgical tools to a specific affected area in the realtime video, and propose a time timer or treatment sequence requiring treatment by using the surgical tools. In this case, the proposed surgical tools, a treatment time timer, a treatment sequence, etc. may be provided in the realtime video through the HMD as AR data.

When the proposal is accepted in the first HMD 100, authentication is performed upon reception of the proposal, and then AR data (text, video, image, voice, etc.) corresponding to the proposal may be tagged to the realtime video to the first HMD 100, and displayed on the second HMD 200.

Here, the authentication may be performed simultaneously on the second HMD 200 that provides the proposal and the first HMD that received the proposal. Referring to FIG. 13, the responsibility setting unit 1130 may receive signatures from the first HMD 100 and the second HMD 200, store the received signatures in advance, and display the signatures on the first HMD 100 and the second HMD 200 to share a signature authentication screen according to the proposal and the proposal acceptance.

As in FIG. 13(a), when a proposal by the collaborating surgeon 20 is received from the second HMD 200, a collaborating surgeon avatar that automatically performs the signature of the preset collaborating surgeon for a proposal responsibility along with the proposal may be provided to the first HMD 100 and the second HMD 200.

Further, as in FIG. 13(b), when the proposal is accepted by the first HMD 100, an operating surgeon avatar that automatically performs a preset operating surgeon signature for the proposal acceptance responsibility may be provided to the first HMD and the second HMD.

Further, when the operating surgeon 10 and the collaborating surgeon 20 set a condition for an automatic signature, the corresponding avatar may also perform the automatic signature. Specifically, the responsibility setting unit 1130 may collect data on the results of a pre-survey conducted on the collaborating surgeon 20 and the operating surgeon 10 or the surgical style of the surgical video recommended by the collaborating surgeon/operating surgeon, and provide avatar attributes (automatic signature condition) corresponding to the collaborating surgeon 20 and the operating surgeon 10, respectively to a terminal (HMD or a user terminal) of each of the collaborating surgeon and the operating surgeon to specify the avatar attribute.

As an example, the collaborating surgeon may recommend pre-recorded other surgical videos before or during the surgery to the operating surgeon and participants (the first assistant and the second assistant). The remote collaborative treatment providing server 1000 may statically extract and determine a surgical style preferred by the collaborating surgeon using the metadata of the recommended surgery videos.

Through this, the operating surgeon may accept the proposal and agree to responsibility without having to individually respond to the acceptance of the proposal that occurs during real-time surgery.

In this case, when the proposal is a proposal of the collaborating surgeon received from the second HMD 200, the tag may contain the responsibility of the operating surgeon 10 wearing the first HMD 100 for accepting the proposal and record the proposal responsibility of the collaborating surgeon 20 wearing the second HMD 200 as responsibility information.

In this case, the proposal may be provided not only to the first HMD 100, but also to the corresponding third HMD 300 based on the role of the first assistant or the second assistant depending on the feature of the proposal.

In addition, the second HMD 200 may directly set a proposal and transmit the proposal to the first HMD 100, or receive a proposal information to be proposed to the first HMD 100 from the AI-based recommendation server 3000 and provide the proposed information to the first HMD 100.

In addition, the first HMD 100 may receive a proposal recommendation (automatic proposal) directly from the AI-based recommendation server 3000.

In this case, the proposal may be made by the tag section information of the tag section, that is, by treatment, surgery, or procedure corresponding to the detailed section, and when similar/same recommended tags are generated, the AI-based recommendation server 3000 may propose optimal tag information according to a preset priority by considering a preset tag importance, treatment/surgery difficulty, etc.

### (First embodiment) Proposal - Second HMD directly proposes to first HMD

The DB 2000 may store surgical videos tagged with at least one tag, and classify and store the surgical videos according to classification criteria such as hospital/doctor, patient status, treatment and surgery category, etc.

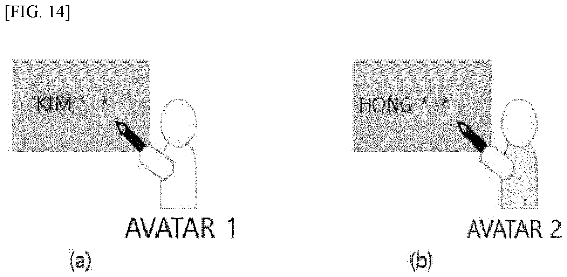

Referring to FIG. 14, the second HMD 200 may selectively output at least one of a first screen (not illustrated) that outputs the realtime video captured by the first HMD 100, and a second screen (in FIG. 14) that displays at least one proposal which may be made during the realtime surgery as a preview window. In this case, the first screen and the second screen may be provided by the collaborative treatment support unit 1100.

The second HMD 200 may load at least one of the tags tagged in the surgery video stored in the DB 2000 as an interested tag into the preview window, and set a display time and a display interval of the interested tag. The collaborative treatment support unit 1100 may output the interested tag to a preview window on the second screen according to the settings of the second HMD 200.

The second HMD 200 may selectively or automatically propose the interested tag depending on the settings to the first HMD 100.

### (Second embodiment) Proposal - Second HMD proposes AI-based recommended proposal to first HMD

The AI-based recommendation server 3000 may extract tags tagged with similar surgeries similar to the realtime surgery from the previous surgery video of the collaborating surgeon, and the tags in the preview window.

Here, the previous surgery video may be a video of a surgery in which the collaborating surgeon participated as the operating surgeon or the collaborating surgeon, a surgery video played by the collaborating surgeon, etc.

The collaborating surgeon may selectively or automatically propose the recommended tag of the preview window to the first HMD 1000 according to settings.

### (Third embodiment) Proposal - Automatically proposing AI-based recommended proposal to first HMD by proposal of collaborating surgeon

When a tag tagged upon a similar surgery to the realtime surgery is not proposed during a current realtime surgery among previous surgery videos which the collaborating surgeon participated from the DB 2000, the AI based recommendation server 3000 may automatically proposing the target tagged upon the similar surgery to the first HMD by the proposal of the collaborating surgeon.

In this case, the AI based recommendation server 3000 may determine a tagging point and a tagging interval of the tag proposed in the realtime video, and automatically propose the tag to the first HMD, based on a tagging point and a tagging interval of the tag tagged upon the similar surgery.

### (Fourth embodiment) Proposal - AI based recommendation proposal corresponding to automatic responsibility agreement setting

The first HMD 100 and the second HMD 200 may automatically set a responsibility agreement for a specific treatment or surgery predefined for each surgery category and patient condition.

The responsibility setting unit 1130 may request a recommendation from the AI-based recommendation server 3000 for a specific treatment or surgery for which responsibility agreement for each HMD is automatically set.

In addition, when a proposal for a specific treatment or surgery predefined for each HMD occurs, the responsibility setting unit 1130 provides the corresponding HMD with an avatar that automatically performs the preset signature of the corresponding HMD for which responsibility agreement is automatically set to perform an authentication.

When a detailed section included in a similar surgery similar to the realtime surgery among surgery videos prestored in the DB 2000 corresponds to a predefined specific treatment or surgery in which the responsibility agreement is set by the second HMD 200, the AI-based recommendation server 3000 may automatically propose tags included in the detailed section to the first HMD 100.

In this case, upon receiving the proposal for the predefined specific treatment or surgery in which the responsibility agreement is automatically set, the tagging unit 1120 may automatically tag to the realtime video.

Further, when a proposal for a specific treatment or surgery predefined for each HMD occurs, the responsibility setting unit 1130 provides the first and second HMDs with an avatar that automatically performs the preset signature of the corresponding HMD for which responsibility agreement is automatically set.

### (Fifth embodiment) Proposal - Automatically proposing required tag for each HMD during surgery

The AI-based recommendation server 3000 may extract a tag requiring essential playback in each HMD, and automatically propose the extracted tag to the corresponding HMD based on role information (the collaborating surgeon, the first assistant, and the second assistant) in this realtime surgery among the wearer's information for each HMD for the realtime surgery.

Meanwhile, in the above description, it is described that tagging responsibility for detailed sections depends on whether the operating surgeon accepts the collaborating surgeon's proposal during the realtime surgery. On the other hand, when the operating surgeon and the collaborating surgeon discuss a surgery responsibility interval in advanced before starting the surgery, the collaborative treatment support unit 1100 may divide the entire surgical section into known treatments in advance and divide the divided surgery sections into detailed sections as illustrated in FIG. 11(a), and provided to the realtime video output to each HMD, and a pre-discussed responsibility party may be matched with the detailed sections corresponding to the known treatments in advance, and tagged.

In this case, it is necessary to determine whether a treatment currently performed by the operating surgeon corresponds to the known treatments in advance, and this may be determined through a model generated by machine-learning of data included in the existing surgeon videos.

As an example, when an entire surgery which is previously discussed is performed in the order of skin incision, blood vessel clamping, and bilateral blood vessel connection, the remote collaborative treatment providing server 1000 may divide each treatment/surgery into detailed sections and provide a real-time video, and when it is determined that the operating surgeon performs the treatment specified in the 'order designated in advance by the collaborating surgeon', the remote collaborative treatment providing server 1000 may provide AR data in which a preset collaborating surgeon's avatar signs the responsibility section in the realtime video.

As such, the remote collaborative treatment providing system of the present invention can extract tags that may be issues in the realtime surgery and tag the tags to the realtime video through the methods of various embodiments as described above. In this case, each embodiment may also be individual or combined.

In particular, when tagging a tag according to the collaborating surgeon's proposal of a surgery progress sequence or specific treatment sequence, information on the responsibility party according to the operating surgeon's acceptance or objection is recorded as tag information, so the responsibility party information may be easily used to determine the responsibility.

Meanwhile, when tagging a real-time video, the tagging unit 1120 may mask and process a part that the patient requests to censor to protect personal information, or replace the corresponding part with another realtime video part of the same time period.

The remote collaborative treatment providing server 1000 may store surgical videos tagged with a plurality of tags during the realtime surgery in the DB 2000. In this case, the stored surgery videos may be provided to be utilized according to the purposes of the operating surgeon, the collaborating surgeon, and an educator/trainee. The remote collaborative treatment providing system of the present invention may be implemented as a surgical video brokerage system that brokers the use of surgical videos through the video brokerage server 4000 and distributes profits according to utilization.

### Brokerage system for transaction and use of surgery video including tags

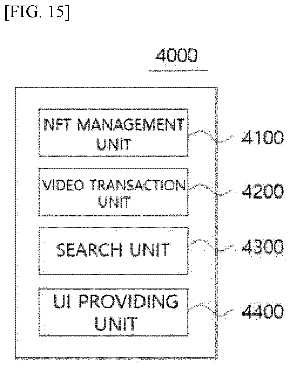

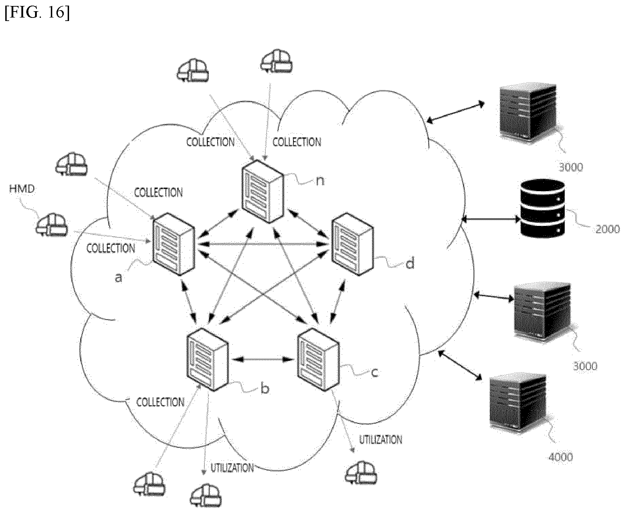

FIG. 15 is a block diagram describing a configuration of a video brokerage server of FIG. 1. FIG. 16 is a diagram describing a blockchain structure of a system for providing a remote collaborative treatment.

Referring to FIG. 15, the video brokerage server 4000 may include an NFT management unit 4100, a video transaction unit 4200, a search unit 4300, and a UI providing unit 4400.

In addition, referring to FIG. 16, the remote collaborative treatment providing system may form a blockchain network with hospital servers a to n of member hospitals, and a surgery video generated during a realtime surgery may be collected through respective hospital servers (a to n), and a surgery video for utilization may be provided. When surgery videos are stored and collected in DB 2000 from the respective hospital servers a to n, the NFT management unit 4100 assigns a unique ID to the collected surgery video to issue a non-fungible token (NFT) based ownership to the corresponding hospital.

The video brokerage server 4000 may manage the surgery video or tags included in the surgical video by settling a usage fee for the sales or use using cryptocurrency when a user terminal of a third-party sells or uses the video.

In addition, the video brokerage server 4000 may issue an NFT ownership for each tag section by assigning a unique ID to each tag section included in the surgery video when a partial sale is requested for a surgery video that has an ownership from a specific hospital server. Accordingly, split sales of surgery or treatment videos for each tag section may be provided.

The NFT management unit 4100 may manage the transfer of NFT ownership to the hospital that sold the NFT ownership once the preset cost is settled when transaction surgery video for each hospital server.

In addition, the NTF management unit 4100 compares the owner of the surgical video requested by the user terminal with the affiliated hospital of the user terminal requesting the use of the surgery video, and if both sides not the same, the NFT management unit 4100 may determine that the user terminal belongs to a third party without ownership, and request the video transaction unit 4200 to calculate the usage fee.

The video transaction unit 4200 may calculate the usage fee based on the number of tags included in the surgery video requested for use by the user terminal and the preset importance of each tag.

Here, the tag may include at least one of tag information, responsibility party information, tag section within the relevant surgery video, tag section information, tag section information inputter, NFT-based ownership information, sales and use history, and token settlement information.

Here, the tag section within the surgery video may be a section divided by detailed treatment or detailed surgery included in the surgery video. Further, the tag section information becomes the detailed treatment or detailed surgery information, and the tag section information inputter may be an operating surgeon or collaborating surgeon who performed the detailed treatment or detailed surgery.

Depending on the use of the user terminal and sales by hospital server, if the information included in the tag is changed or new information is generated, the blockchain according to the updated information may be added and updated to the distribution ledger through consensus of nodes in the blockchain network.

In addition, the video transaction unit 4200 may accumulate cryptocurrency to the tag section information inputter based on the usage fee collected by the user terminal when using the tag section information in a specific user terminal.

The search unit 4300 provides a search service according to the purpose of video playback based on the tag information and tag section information of the tag stored in the DB 2000, allowing the user to utilize the necessary surgical videos and tags for each user terminal.

Here, the user terminal is an HMDs 100, 100-1, 100-2, 200, and 500 worn by at least one of the operating surgeon performing the realtime surgery, the collaborating surgeon collaborating with the realtime surgery, and at least one assistant and educator/trainee participating in the realtime surgery.

When receiving participating surgery information from the user terminal, the AI-based recommendation server 3000 may recommend and provide surgery videos and tags for similar surgeries.

In addition, when the AI-based recommendation server 3000 receives user role information for participating surgeries from the user terminal, the AI-based recommendation server 3000 may recommend tags included in similar surgeries as required playback tags based on the user role information.

The UI providing unit 4400 may provide to the corresponding user terminal a list of plurality of tags included in similar surgeries recommended to the user terminal by the AI-based recommendation server 3000 for selection to be used or removed according to the purpose of use.

The UI providing unit 4400 may preset required playback tag information for each user terminal based on the role information of the user terminal, and provide tags corresponding to the preset required playback tag information by excluding the tags from the list.

In this case, the video transaction unit 4200 may calculate and settle the usage fee by reflecting the number of tags used or removed by the corresponding user terminal.

As described above, the brokerage system of the present invention brokers use and transactions by assigning an NFT-based ownership to the surgery video containing tags to broker transparent and reliable transactions, and distribute transaction profits.

In addition, through AI-based recommendations, an optimal surgical video and optimal tags can be provided according to a playback purpose of a user (operating surgeon, collaborating surgeon, first assistant, second assistant, or educator/trainee), and the utilization of the surgical video can be increased.

The embodiments of the present invention are implemented in a form of a program command which may be performed through various computer means and may be recorded in the computer readable medium. The computer readable medium may include a program command, a data file, a data structure, etc., singly or combinationally.

The program command recorded in the medium may be specially designed and configured for the present invention, or may be publicly known to and used by those skilled in the computer software field. An example of the computer readable recording medium includes a magnetic media, such as a hard disk, a floppy disk, and a magnetic tape, optical media such as a CD-ROM and a DVD, magneto-optical media such as a floptical disk, and all types of hardware devices such as a ROM, a RAM, and a flash memory, which are specially configured to store and execute the program command. An example of the program command includes a high-level language code executable by a computer by using an interpreter and the like, as well as a machine language code created by a compiler.

The hardware device may be configured to be operated with one or more software modules in order to perform the operation of the present invention and vice versa.

Although the embodiments of the present invention have been described in detail with reference to the accompanying drawings, the present invention is not limited thereto and may be modified in many different forms without departing from the technical concept of the present invention.

Accordingly, the various embodiments disclosed in the present invention are not intended to limit the technical spirit but describe the present invention and the technical spirit of the present invention is not limited by the following embodiments.

Therefore, it should be understood that the above-described embodiments are illustrative in all aspects and do not limit the present invention. The protection scope of the present invention should be construed based on the following appended claims and it should be appreciated that the technical spirit included within the scope equivalent to the claims belongs to the scope of the present invention.

## Claims

1. A system for providing a remote collaborative treatment, the system comprising:
a first HMD worn by an operating surgeon at an actual surgery site, and including a camera capturing a gaze area of the operating surgeon and a surrounding area, and a display unit outputting a realtime video captured by the camera;
a second HMD worn by a collaborating surgeon positioned remotely from the operating surgeon, receiving and outputting the realtime video captured by the first HMD, and receiving a proposal for a surgery progress sequence or a specific treatment sequence include in a surgery by the collaborating surgeon; and
a remote collaborative treatment providing server providing a UI for selecting whether to accept the proposal to the first HMD upon receiving the input proposal from the second HMD, and generating a tag according to whether the operating surgeon accepts the proposal and tagging the generated tag to the realtime video,
wherein the tag includes responsibility party information for a realtime surgery according to the proposal.

2. The system for providing a remote collaborative treatment of claim 1, wherein the tag further includes tagging location information in the realtime video, and the information included in the tag is stored in a blockchain-based distribution ledger.

3. The system for providing a remote collaborative treatment of claim 2, wherein when receiving a preset specific input from the first HMD, the remote collaborative treatment providing server generates a timestamp and tags the timestamp to the realtime video, and divides the real-time surgery into a plurality of detailed sections defined by the operating surgeon based on a plurality of tagged timestamps, and
the tagging location information includes a start timestamp and an end timestamp of a detailed section including a tagging point of the corresponding tag.

4. The system for providing a remote collaborative treatment of claim 3, wherein the preset specific input becomes at least one of a gesture, a voice command, and a control signal command, and
the detailed section is a section corresponding to a detailed treatment and a surgery sequence of the realtime surgery defined by the operating surgeon.

5. The system for providing a remote collaborative treatment of claim 1, wherein the remote collaborative treatment providing server provides a preview of the proposal to the first HMD jointly with the UI, and inserts AR data corresponding to the proposal into the realtime video, and displays the realtime video inserted with the AR data to the first HMD and the second HMD when the proposal is accepted through the first HMD..

6. The system for providing a remote collaborative treatment of claim 1, wherein the remote collaborative treatment providing server
provides, to the first HMD and the second HMD, an avatar of a collaborating surgeon which automatically performs a signature of a preset collaborating surgeon for a proposal responsibility jointly with the proposal when the proposal by the collaborating surgeon is received from the second HMD, and
provides, to the first HMD and the second HMD, an avatar of an operating surgeon automatically performing a preset operating surgeon signature for a proposal acceptance responsibility when accepting the proposal in the first HMD.

7. The system for providing a remote collaborative treatment of claim 1, wherein the remote collaborative treatment providing server
is automatically set with a responsibility agreement for a specific treatment or surgery predefined for each surgery category and patient condition from each of the first HMD and the second HMD, and
provides the first and second HMDs with an avatar that automatically performs the preset signature of the corresponding HMD for which responsibility agreement is automatically set when a proposal for a specific treatment or surgery predefined for each HMD occurs.

8. The system for providing a remote collaborative treatment of claim 7, further comprising:
a DB storing a surgery video tagged with at least one tag; and
an AI-based recommendation server automatically proposing tags included in the detailed section to the first HMD when a detailed section included in a similar surgery similar to the realtime surgery among surgery videos prestored in the DB corresponds to a predefined specific treatment or surgery in which the responsibility agreement is set by the second HMD.

9. The system for providing a remote collaborative treatment of claim 7, wherein the remote collaborative treatment providing server automatically tags, when receiving the proposal for the predefined specific treatment or surgery in which the responsibility agreement is automatically set from the first HMD, the proposal to the realtime video.

10. The system for providing a remote collaborative treatment of claim 7, further comprising:
an AI-based recommendation server automatically proposing, when a detailed section included in a similar surgery similar to the realtime surgery among prestored surgery videos corresponds to the predefined specific treatment or surgery in which the responsibility agreement is set by the first HMD, at least one tag included in the detailed section of the similar surgery to the first HMD,
wherein the remote collaborative treatment providing server tags at least one tag automatically proposed by the AI-based recommendation server to the realtime video.

11. The system for providing a remote collaborative treatment of claim 1, wherein the second HMD selectively outputs at least one of a first screen that outputs the realtime video captured by the first HMD, and a second screen that displays at least one proposal which may be made during the realtime surgery as a preview window.

12. The system for providing a remote collaborative treatment of claim 11, further comprising:
a DB classifying and storing the surgery video tagged with at least one tag for each doctor, for each patient condition, and for each treatment and surgery category,
wherein the second HMD sets at least one of the tags tagged to the surgery video stored in the DB as an interested tag, and is set with a display time and a display interval of the interested tag, and
the remote collaborative treatment providing server outputs the interested tag to a preview window of the second screen according to settings of the second HMD.

13. The system for providing a remote collaborative treatment of claim 11, further comprising:
the AI-based recommendation server recommending the tags tagged to the similar surgery similar to the realtime surgery as the preview window from a previous surgery video of the collaborating surgeon.

14. The system for providing a remote collaborative treatment of claim 1, further comprising:
an AI-based recommendation server automatically providing, when the tag tagged upon the similar surgery similar to the realtime surgery is not proposed during the realtime surgery among the previous surgery videos performed by the collaborating surgeon, the tag tagged upon the similar surgery to the first HMD by the proposal of the collaborating surgeon.

15. The system for providing a remote collaborative treatment of claim 14, wherein the AI-based recommendation server determines a tagging point and a tagging interval of the tag proposed in the realtime video, and automatically proposes the tag to the first HMD, based on a tagging point and a tagging interval of the tag tagged upon the similar surgery.

16. The system for providing a remote collaborative treatment of claim 1, wherein based on third HMDs worn by a plurality of assistants, respectively during the realtime surgery at the actual surgery site, a DB storing the surgery video tagged with at least one tag, and role information of a wearer for each HMD for the realtime surgery, a tag requiring essential playback is extracted in each HMD, and automatically proposed to a corresponding HMD.
